# EUROPÄISCHE PATENTSCHRIFT

(11) **EP 2 126 110 B1**
(45) Veröffentlichungstag und Bekanntmachung des Hinweises auf die Patenterteilung: **25.12.2013**
(21) Anmeldenummer: 07800192.2
(22) Anmeldetag: 26.09.2007
(51) Int. Cl.: C12Q 1/48

(54) **MESSUNG VON DER AKTIVITÄT EINES KYNURENIN-UMWANDELNDEN ENZYMS UND/ODER EINES KYNURENSÄURE-, ANTHRANILSÄURE- UND/ODER 3-HYDROXYKYNURENIN-ERZEUGENDEN ENZYMS**
MEASUREMENT OF THE ACTIVITY OF A KYNURENINE-CONVERTING ENZYME AND/OR OF A KYNURENIC ACID, ANTHRANILIC ACID AND/OR 3-HYDOXYKYNURENINE-PRODUCING ENZYME
MESURE DE L'ACTIVITÉ D'UNE ENZYME TRANSFORMANT DE LA KYNURÉNINE ET/OU D'UNE ENZYME PRODUISANT DE L'ACIDE KYNURÉNIQUE, DE L'ACIDE ANTHRANILIQUE ET/OU DE LA 3-HYDROXYKYNURÉNINE

(30) Priorität: 27.03.2007 AT 19507 U
(43) Veröffentlichungstag der Anmeldung: 02.12.2009
(73) Patentinhaber: Kepplinger, Berthold, 3300 Amstetten (AT); Baran, Halina, 1210 Wien (AT)
(72) Erfinder: BARAN, Halina, A-1210 Wien (AT)
(74) Vertreter: Sonn & Partner Patentanwälte
(86) Internationale Anmeldenummer: PCT/AT2007/000452
(87) Internationale Veröffentlichungsnummer: WO 2008/116235

(56) Entgegenhaltungen:
- WO-A-96/01893
- WO-A-99/58968
- WO-A-2006/105907
- WO-A-2006/124892
- US-B1- 6 265 442
- KEPPLINGER B ET AL: "Cerebrospinal fluid of multiple sclerosis patients exert significantly weaker inhibition of kynurenine aminotransferase I activity in rat liver homogentate" MULTIPLE SCLEROSIS, BASINGSTOKE, GB, Bd. 12;S1-S228, 2006, Seite 496, XP008087035 ISSN: 1352-4585
- BARAN H ET AL: "Lowered kynurenine aminotransferases activities in CNS of MS patients" SOCIETY FOR NEUROSCIENCE ABSTRACTS, Bd. 26, Nr. 1-2, 2000, Seiten Abstract No.-481.12, XP002463565 & 30TH ANNUAL MEETING OF THE SOCIETY OF NEUROSCIENCE; NEW ORLEANS, LA, USA; NOVEMBER 04-09, 2000 ISSN: 0190-5295 & http://sfn.scholarone.com/itin2000/main.ht ml?new_page_id=76&abstract_id=18641&is_tec h=0
- NARITSIN D B ET AL: "METABOLISM OF L-TRYPTOPHAN TO KYNURENATE AND QUINOLINATE IN THE CENTRAL NERVOUS SYSTEM: EFFECTS OF 6-CHLOROTRYPTOPHAN AND 4-CHLORO-3-HYDROXYANTHRANILATE" JOURNAL OF NEUROCHEMISTRY, NEW YORK, NY, US, Bd. 65, Nr. 5, 1995, Seiten 2217-2226, XP001007947 ISSN: 0022-3042
- HEYES M P ET AL: "KYNURENINE PATHWAY METABOLITES IN CEREBROSPINAL FLUID AND SERUM IN COMPLEX PARTIAL SEIZURES" EPILEPSIA, RAVEN PRESS LTD., NEW YORK, US, Bd. 35, Nr. 2, März 1994 (1994-03), Seiten 251-257, XP000996552 ISSN: 0013-9580
- HEYES M P ET AL: "QUINOLINIC ACID AND KYNURENINE PATHWAY METABOLISM IN INFLAMMATORY AND NON-INFLAMMATORY NEUROLOGICAL DISEASE" BRAIN, OXFORD UNIVERSITY PRESS, OXFORD, GB, Bd. 115, Nr. PART 5, Oktober 1992 (1992-10), Seiten 1249-1273, XP008064389 ISSN: 0006-8950
- OKUNO E ET AL: "MEASUREMENT OF RAT BRAIN KYNURENINE AMINOTRANSFERASE AT PHYSIOLOGICAL KYNURENINE CONCENTRATIONS" JOURNAL OF NEUROCHEMISTRY, NEW YORK, NY, US, Bd. 57, 1991, Seiten 533-540, XP000654266 ISSN: 0022-3042

## Beschreibung

Das Enzym Kynurenin-Aminotransferase (abgekürzt als KAT) katalysiert die Biosynthese von Kynuren-Säure (KYNA) aus Kynurenin. Mehrere Enzyme an der Peripherie sind für die KYNA-Bildung verantwortlich, und die Rattenleber enthält die höchsten KAT-Aktivitäten (1). Die menschliche ZSF (Zerebrospinalflüssigkeit) und das Serum enthalten geringe oder nicht einmal nachweisbare KAT-Aktivitäten (2). Die Veränderung des Kynurenin-Metabolismus wurde bei neuroimmunologischen, neuroinflammatorischen und neurodegenerativen Prozessen, einschließlich Schizophrenie und Depression, dokumentiert. Neue klinische Marker, die sich auf den Kynurenin-Metabolismus beziehen, sind bei den erwähnten Krankheiten von besonderem Interesse.

WO 2006/124892 betrifft Krankheiten, die im Zusammenhang mit einer Überexprimierung von Alpha-Synuclein stehen, wie beispielsweise die Parkinson'sche Krankheit oder die Alzheimer'sche Krankheit. Mit dem Alpha-Synuclein-Stoffwechsel wird eine Reihe an Enzymen in Verbindung gebracht, darunter KAT II. WO 2006/105907 betrifft die Messung der Konzentration von Tryptophan sowie dessen Abbauprodukte, insbesondere Kynurenin, und weiterer Abbauprodukte wie 3-Hydroxykynurenin und Kynurensäure in Blut, Urin, Speichel und CSF zur Diagnose von psychiatrischen Zuständen, wie Depressionen.

US-B1-6 264 442 befasst sich mit Huntington's Disease (HD), welche dazu führt, dass im Gehirn die Kynureninsäure-Produktion reduziert wird und auch zu einem geringeren Anteil an das CSF abgegeben wird.

WO 99/58968 betrifft Abbauprodukte der Kynurenin-Metabolismus in der Diagnose von Epilepsie.

WO 96/01893 betrifft die Klonierung einer Kynurenin-Aminotransferase (KAT).

Baran et al. (11) beschreibt Untersuchungen zum Metabolismus von Kynurensäure. Es wird angegeben, dass die Kynurensäure in Rattenhirnen erzeugt wird und der weitere Metabolismus der Kynurensäure im CSF und Serum von Patienten mit Multipler Sklerose untersucht wurde.

Naritsin et al. (12) beschreibt die Messung der Kynurenin-A-minotransferasen I und II Produkte in Homogenaten von ausgewählten Zellen.

Heyes et al. (13) beschreibt die Messung von Kynurenin-Metaboliten in CSF betrifft.

Heyes et al. (14) betrifft die Messung von Konzentrationen der Metaboliten des Kynurenin-Metabolismus wie beispielsweise L-Kynurenin und Kynurensäure, welche bei Huntington's Disease oder der Alzheimer'schen Krankheiten reduziert sind.

Okuno et al. (15) betrifft die Aktivitätsmessung einer Kynurenin-Aminotransferase.

Kepplinger et al. (3) offenbart die Bestimmung der KAT-Aktivität in Anwesenheit von Zerebrospinal-Flüssigkeiten als interferierende Probe bei Multipler Sclerose. Es besteht das Ziel weitere Verbesserungen bzw. neue diagnostische Proben aufzufinden.

Daher sieht die vorliegende Erfindung ein Verfahren zur Messung der Aktivität einer Kynurenin-Aminotransferase (KAT), umfassend den Schritt des Messens der Aktivität in Anwesenheit einer interferierenden Probe ausgewählt aus einer Serumprobe, und der Detektion der Umwandlung von Kynurenin und/oder Kynurensäure und/oder Anthranilsäure und/oder 3-Hydroxykynurenin. Weiters betrifft die Erfindung ein Verfahren zur Messung der Aktivität einer Kynurenin-Aminotransferase (KAT), umfassend den Schritt des Messens der Aktivität in Anwesenheit einer interferierenden Probe, vorzugsweise ausgewählt aus einer ZSF (Zerebrospinal-Flüssigkeit) oder Serumprobe, und der Detektion der Umwandlung von Kynurenin und/oder Kynurensäure und/oder Anthranilsäure und/oder 3-Hydroxykynurenin und/oder Pyridoxal-5'-phosphat, dadurch gekennzeichnet, dass die Aktivität bei zwei unterschiedlichen Mengen der interferierenden Probe bestimmt wird, und ein Verhältnis dieser Aktivitäten bestimmt wird. Weitere Aspekte der Erfindung sind in den Ansprüchen definiert. In Körperflüssigkeiten wie z.B. ZSF und/oder Serum sind Anteile vorhanden, die mit der Aktivität der Kynurenin-Umwandlung interferieren. Dieser interferierende Effekt wird bei Patienten mit mehreren Erkrankungen verringert (oder erhöht). Ein Vergleich von zwei Effekten, die von zwei verschiedenen Dosen einer interferierenden Probe gezeigt wurden, vorzugsweise ausgewählt aus ZSF und/oder Serum, sehen ein Verhältnis R_{HB} oder R_{BK} vor, welches mit der Pathologie/Krankheit in Verbindung steht. Daher kann das erfindungsgemäße Verfahren für Diagnostika, wie nachstehend beschrieben, verwendet werden. Vorzugsweise wird eines oder beides, die Reduktion von Kynurenin und/oder von Pyridoxal-5'-phosphat und/oder die Bildung von Kynurensäure, Anthranilsäure und/oder 3-Hydroxykynurenin nachgewiesen.

Das Enzym ist eine Kynurenin-Aminotransferase (KAT), vorzugsweise KAT I, KAT II oder KAT III. Genannt wird auch die Verwendung jeder isolierten oder synthetisierten Transferase, die eine Ähnlichkeit mit KAT I, KAT II oder KAT III aufweist.

Vorzugsweise stammt die Aktivität der KAT von einer Gewebeprobe, vorzugsweise einer Lebergewebeprobe, vorzugsweise einem Gewebshomogenat, mehr bevorzugt einer isolierten oder synthetisierten Gewebeprobe. KAT ist ein endogenes Enzym, das in vielen Geweben vorhanden ist, und kann in ungereinigtem oder marginal gereinigtem Zustand verwendet werden, wie im Zusammenhang mit einer Gewebeprobe oder einem Homogenat. Eine solche Gewebeprobe stammt vorzugsweise von einem Säuger, vorzugsweise einem Nagetier, z.B. einer Ratte, oder von einem Menschen.

Bei den am meisten bevorzugten Ausführungsformen stammt die interferierende Probe, vorzugsweise eine ZSF-Probe und/oder Serum, von einem Säuger, vorzugsweise von einem Menschen. Die interferierende Probe kann von einem gesunden Probanden stammen, die als Standard-Referenz verwendet werden kann, oder von einem Probanden mit einer Krankheit, von welcher erwartet wird, dass sie eine geringere Inhibition oder Aktivierung der Kynurenin-Umwandlung zeigt. Es ist auch möglich, verschiedene Mengen der interferierenden Probe, vorzugsweise ZSF und/oder Serum, zu verwenden und eine Interferenz-Kurve in Abhängigkeit der Menge der interferierenden Probe oder des Enzyms zu konstruieren. Auch ohne Zeichnung einer vollständigen Kurve ist es möglich, zwei bestimmte Mengen der interferierenden Probe (oder des Enzyms) auszuwählen und das Verhältnis der geoffenbarten unterschiedlichen Auswirkungen auf die Umwandlungen zu bestimmen. Diese Verhältnisse (R_{HB} und R_{BK}) können verwendet werden, um eine bestimmte Krankheit zu diagnostizieren (z.B. liegt R_{HB} zwischen 1,5 und 3,5 und R_{BK} zwischen 0 und 2,5).

Vorzugsweise umfasst das Verfahren den Schritt des Vergleichens der Aktivität mit der Aktivität der KAT, vorzugsweise von einer Gewebeprobe, in Abwesenheit der interferierenden Probe oder durch Verwendung verschiedener Mengen der interferierenden Probe oder des Enzyms.

In einem weiteren Aspekt sieht die vorliegende Erfindung ein Verfahren zur Diagnostizierung einer Pathologie vor, die mit dem Kynurenin- oder Kynurensäure-Metabolismus in Verbindung steht, unter Verwendung des (in vitro)-Verfahrens, wie oben beschrieben, wobei eine Verringerung der Aktivität um weniger als 80%, vorzugsweise um weniger als 60%, besonders bevorzugt um weniger als 50%, mehr bevorzugt um weniger als 40%, insbesondere bevorzugt um weniger als 30%, am meisten bevorzugt um weniger als 20%, im Vergleich mit der Aktivität ohne die interferierende Komponente (Kontrolle) die Pathologie anzeigt. Das Verhältnis der Effekte verschiedener Mengen an interferierender Probe, vorzugsweise ausgewählt aus ZSF und/oder Serum, kann für ein Verfahren zur Diagnostizierung verwendet werden.

Bei besonderen Ausführungsformen ist die Pathologie eine neuroimmunologische, neuroinflammatorische oder neurodegenerative Pathologie, insbesondere Schizophrenie, Depression oder Multiple Sklerose (MS).

In besonderen Ausführungsformen wird als interferierende Probe eine Serumprobe eingesetzt. Überraschenderweise hat sich gezeigt, dass ähnliche inhibitorische Eigenschaften der ZSF auch in einfacher zu Handhabenden Serumproben möglich ist. Unter Serum wird gemäß der vorliegen Erfindung sämtliche Serum-umfassenden Körperflüssigkeiten verstanden, inklusive Blut (mit zellulären Bestandteilen) oder Blutplasma (mit Gerinnungsfaktoren), wobei Serum am meisten bevorzugt ist.

Gemäß einem anderen Aspekt sieht die vorliegende Erfindung ein Set, welches eine biologische Probe, die KAT, vorzugsweise in Verbindung mit einer Gewebeprobe oder einem Homogenat, insbesondere einem Leberhomogenat, geeigneten Puffern und Kynurenin, vorzugsweise L-Kynurenin aufweist, und gegebenenfalls auch Pyridoxal-5'-Phosphat umfasst, vor.

Das Set kann für die erfindungsgemäßen Verfahren verwendet werden. Die Enzyme können vorzugsweise auch in Form einer synthetisierten Leber oder einem Homogenat vorliegen, welches eine Ähnlichkeit mit KAT I, KAT II oder KAT III oder Aminotransferase(n) mit ähnlichen Eigenschaften aufweist.

Im Set ist das Enzym eine Kynurenin-Aminotransferase (KAT), vorzugsweise KAT I, KAT II oder KAT III.

Außerdem weist das Set vorzugsweise eine Oxo-Säure auf, vorzugsweise ausgewählt aus Pyruvat, 3-Hydroxypyruvat, 2-Oxoglutarat, 2-Oxoisovalerat, 2-Oxoadipat, Phenylpyruvat, 2-Oxobutyrat, Glyoxalat, Oxaloacetat, 2-Oxo-gamma-Methiolbutyrat, 2-Oxo-n-valerat, 2-Oxo-n-caproat und 2-Oxoisocaproat.

Ebenfalls bevorzugt kann das Set ein Protein-denaturierendes Mittel, vorzugsweise in einem Mikrozentrifugenröhrchen, aufweisen.

Zum Vergleich von Messungen umfasst das Set vorzugsweise L-Kynurenin-, Pyridoxal-5'-phosphat-, Kynurensäure-, Anthranilsäure- und/oder 3-Hydroxykynurenin-Standards.

Die vorliegende Erfindung ist durch die folgenden Beispiele näher veranschaulicht, ohne jedoch auf diese eingeschränkt zu sein.

### Beispiele:

### Beispiel 1: Messung der KATs (KAT I, KAT II und KAT III)-Aktivitäten in der Leber in Anwesenheit von ZSF und Serum zeigt signifikant verringerte KAT-Aktivitäten.

ZSF und Serum verringerten die KYNA-Bildung (KAT I-Aktivität) im Rattenleber-Homogenat signifikant um 70% (30% der Kontrolle) und die KAT II-Aktivität von Leber-Homogenat wurde moderat durch humanes ZSF oder Serum beeinflusst. Zwei verschiedene Mengen von jeweils ZSF oder Serum wurden als Komposition der Mischung in der KAT-Reaktion für das Diagnostikum aufgebracht, und ein Verhältnis beider Effekte wird erstellt. Humane ZSF oder Serum vom Kontroll-Probanden und von einem MS-Patienten zeigen einen unterschiedlichen Effekt auf die Leber-KAT I-Aktivität, d.h. auf die Bildung von KYNA und anderen Kynurenin-Metaboliten wie Anthranilsäure und 3-Hydroxykynurenin.

ZSF oder Serum von MS-Patienten zeigt eine signifikant schwächere Fähigkeit, die KAT I-Aktivität im Leber-Homogenat zu senken (60% der Kontrolle), d.h. eine signifikant höhere Bildung von KYNA, im Vergleich zur Wirkung von ZSF oder Serum von Kontroll-Probanden, welche Hemmung von KAT I 20-30% der Kontrolle betrug (3). Die KAT II-Aktivität von Rattenleber wurde durch humane ZSF oder Serum moderat beeinflusst.

### Beispiel 2: KAT-Test

Der KAT-Test ist allgemein bekannt und wurde gemäß der veröffentlichten Arbeit (Baran et al., 2004) durchgeführt. (Die KAT-Aktivitäts-Messung wurde ebenfalls 1994; 2000; 2004 veröffentlicht.) Für den diagnostischen Zweck enthielt der Reaktionscocktail eine Mischung aus einem Rattenleber-Homogenat und ZSF oder Serum. L-Kynurenin, Pyruvat, Pyridoxal-5'-phosphat und 150 mM 2-Amino-2-methyl-1-propanol(AMPOL)-Puffer, pH 9,6, für KAT I oder 150 mM Tris-Acetat-Puffer, pH 7,0, für KAT II in einem Gesamtvolumen von 0,2 ml. Nach Inkubation (16 h lang; die Zeit ist variabel) bei 37°C wurde die Reaktion durch Zugabe von 10 µl 50% TCA bestimmt. Danach wurde 1 ml 0,1 M HCl zugegeben, und denaturiertes Protein wurde mittels 10 min bei 14 000 U/min (Eppendorf Microfuge) entfernt. Der Zustand des Substrats L-Kynurenin, Pyruva und Pyridoxal-5'-phosphat sind ebenfalls variabel und können gemäß bereits veröffentlichter Arbeiten (1, 2, 3) verwendet werden.

Die Messung der KAT-Aktivität, und/oder der Kynurenin- und/oder der Pyridoxal-5'-phosphat-Konzentration und/oder der Kynurenin-Metaboliten, d.h. die Bildung von KYNA, Anthranilsäure und/oder von 3-Hydroxykynurenin, kann mit verschiedenen Methoden durchgeführt werden:
1. Test mittels Spektrophotometer, wie von Baran et al., 1994 (5) beschrieben;
2. Test mittels-HPLC und Anthranilsäure, wie von Baran et al., 1995 (8) und ebenso (6, 7) beschrieben;
3. Test mittels radioenzymatischer Methode, wie von Kepplinger et al., 2005 (3) beschrieben.

Ad 1) Die neu gebildete KYNA wurde spektrophotometrisch bei 333 nm (Knox, 1953) bestimmt.

Ad 2) Messung der KYNA mittels HPLC erfolgte gemäß Shibata, 1988 (9) und Swartz, 1990 (10) mit einer von Baran et al., 1996, beschriebenen Modifikation. Der erhaltene Überstand wird auf eine Dowex 50 W-Kationenaustauschersäule aufgetragen, und KYNA wird mit 2 ml destilliertem Wasser, wie von Turski et al., 1989 (11) beschrieben, eluiert und mittels HPLC, gekoppelt mit Fluoreszenz-Detektion (Shibata et al., 1988; Swartz et al., 1990) bestimmt. Das HPLC-System, welches für die Analyse von KYNA und Anthranilsäure bzw. 3-Hydroxykynurenin verwendet wurde, bestand aus Folgendem: Pumpe (Shimadzu, LC-6A), Fluoreszenz-Detektor (Shimadzu, RF-535), eingestellt mit einer Anregungswellenlänge von 340 nm und einer Emissionswellenlänge von 398 nm, und einem Shimadzu C-R5A Chromatopac Integrator. Die mobilde Phase (isokratisches System) besteht aus 50 mM Natriumacetat, 250 mM Zinkacetat und 4% Acetonitril, pH 6,2, und wird durch eine 10 cm x 0,4 cm-Säule (HR-80, C-18, Teilchengröße 3 |o,M, In-Chrom, Österreich) mit einer Fließgeschwindigkeit von 1,0 ml/min, die bei Raumtemepratur (23°C) laufen lassen wurde, gepumpt. Die Retentionszeit von Anthranilsäure und KYNA ist etwa 3,5 min respektive 5 min mit einer Sensibilität von 250 fmol und 150 fmol pro Injektion (Signal/Rausch-Verhältnis=5).

Ad 3) Die radioenzymatische Methode kann gemäß der von Baran et al., 2004, und Kepplinger et al., 2005, beschriebenen Methode durchgeführt werden.

### Literaturstellen:

1.Okuno E, Nishikawa T, M Nakamura, (1996) Kynurenine aminotransferases in the rat. Localization and Characterization. Recent Advances in Tryptophan Research, edited by Graziella Allegri Filipini et al., Plenum Press, New York, 1996.
2.B. Kepplinger, H. Baran, A. Kainz, H. Ferraz-Leite, J. Newcombe und P. Kalina (2005) Age-related increase of kynurenic acid in human cerebrospinal fluid: Positive correlation with IgG and Beta-microglobulin changes. Neurosignals, 14(3), 126-135.
3.Kepplinger B, Baran H, Kainz A, Zeiner D, Wallner J (2006) Cerebrospinal Fluid of Multiple Sclerosis patients exert significantly weaker inhibition of Kynurenine Aminotransferase I activity in rat liver homogenate. Multiple Sclerosis 2006; 12:S1-S228, P496
4.H. Baran, B. Kepplinger, M. Draxler und H. Ferraz-Leite (2004) Kynurenic acid metabolism in rat, piglet and human tissues. In European Society for Clinical Neuropharmacology by ed L. Battistin, International Proceedings MEDIMOND S.r.1. E505R9004, 227-231.
5.H. Baran, E. Okuno, R. Kido und R. Schwarcz (1994) Purification and characterization of kynurenine aminotransferase I from human brain. J. Neurochem., 62, 730-738.
6.H. Baran, J.A. Hainfellner, B. Kepplinger. P.R. Mazal, H. Schmid und H. Budka (2000) Kynurenic acid metabolism in the brain of HIV-1 infected patients. J. Neural. Transm., 107, 1127-1138.
7.Baran H, Gramer M, Hönack D und W. Löscher Systemic administration of kainate induces marked increase of endogenous kynurenic acid in various brain regions and plasma of rats. Eur J Pharmacol 1995; 286: 167-175.
8.Shibata K. Fluorimetric microdetermination of kynurenic acid, an endogenous blocker of neurotoxicity, by high performance liquid chromatography. J Chromat 1988; 430: 376-380.
9.Swartz KJ, Matson WR, MacGarvey U, Ryan EA, Beal MF. Measurement of kynurenic acid in mammalian brain extracts and cerebrospinal fluid by high-performance liquid chromatography with fluorometric and coulometric electrode array detection. Anal Biochem 1990; 185: 363-376.
10. Turski WA, Gramsbergen JBP, Traitler H, Schwarcz R. Rat brain slices produce and liberate kynurenic acid upon expose to L-kynurenine. J Neurochem 1989; 52: 1629-1636.
11. Baran H et al., Society for Neuroscience Abstracts 26 (1-2) (2000): No.-481.12.
12. Naritsin B et al., Journal of Neurochemistry 65 (5) (1995): 2217-2226.
13. Heyes P et al., Epilepsia 35 (2) (1994): 251-257.
14. Heyes P et al., Brain 115 (Part 5) (1992): 1249-1273.
15. Okuno E et al., Journal of Neurochemistry 57 (1991): 533-540.

## Patentansprüche

1. Verfahren zur Messung der Aktivität einer Kynurenin-Aminotransferase (KAT), umfassend den Schritt des Messens der Aktivität in Anwesenheit einer interferierenden Probe ausgewählt aus einer Serumprobe, und der Detektion der Umwandlung von Kynurenin und/oder Kynurensäure und/oder Anthranilsäure und/oder 3-Hydroxykynurenin oder Pyridoxal-5'-phosphat.

2. Verfahren zur Messung der Aktivität einer Kynurenin-Aminotransferase (KAT), umfassend den Schritt des Messens der Aktivität in Anwesenheit einer interferierenden Probe, vorzugsweise ausgewählt aus einer ZSF (Zerebrospinal-Flüssigkeit) oder Serumprobe, und der Detektion der Umwandlung von Kynurenin und/oder Kynurensäure und/oder Anthranilsäure und/oder 3-Hydroxykynurenin oder Pyridoxal-5'-phosphat, **dadurch gekennzeichnet, dass** die Aktivität bei zwei unterschiedlichen Mengen der interferierenden Probe bestimmt wird, und ein Verhältnis dieser Aktivitäten bestimmt wird.

3. Verfahren nach Anspruch 1 oder 2, **dadurch gekennzeichnet, dass** das die Kynurenin-Aminotransferase (KAT) KAT I, KAT II oder KAT III ist.

4. Verfahren nach Anspruch 1, 2 oder 3, **dadurch gekennzeichnet, dass** die Aktivität von KAT und/oder KAT einer Gewebeprobe, insbesondere ein Gewebe-Homogenat, vorzugsweise einer Lebergewebeprobe, mehr bevorzugt einer isolierten oder synthetisierten Lebergewebeprobe, stammt.

5. Verfahren nach Anspruch 4, **dadurch gekennzeichnet, dass** die Gewebeprobe von einem Säuger, vorzugsweise einem Nagetier oder einem Menschen stammt.

6. Verfahren nach einem der Ansprüche 1 bis 5, **dadurch gekennzeichnet, dass** die interferierende Probe von einem Säuger, vorzugsweise von einem Menschen, stammt.

7. Verfahren nach einem der Ansprüche 1 bis 6, umfassend den Schritt des Vergleichens der Aktivität mit der Aktivität der KAT, vorzugsweise von einer Gewebeprobe, wie in Anspruch 4 oder 5 beschrieben, in Abwesenheit der interferierenden Probe oder durch Verwendung einer anderen Menge der interferierenden Probe oder des Enzyms.

8. Verfahren zur Diagnostizierung einer Pathologie, die mit dem Kynurenin- oder Kynurensäure-Metabolismus in Verbindung steht, vorzugsweise einer neuroimmunologischen, neuroinflammatorischen oder neurodegenerativen Pathologie, Schizophrenie, Depression oder Multiple Sklerose, unter Verwendung des (in vitro-)Verfahrens der Ansprüche 1 bis 7, wobei eine Reduktion der Aktivität um weniger als 80%, vorzugsweise um weniger als 60%, besonders bevorzugt um weniger als 50%, mehr bevorzugt um weniger als 40%, besonders bevorzugt um weniger als 30%, am meisten bevorzugt um weniger als 20%, im Vergleich zur Aktivität ohne die interferierende Komponente (Kontrolle) die Pathologie anzeigt.

9. Verfahren nach einem der Ansprüche 1 bis 8, zur Messung der Aktivität einer KAT, **dadurch gekennzeichnet, dass** die Umwandlung von Kynurenin und/oder Kynurensäure detektiert wird.

10. Verfahren nach einem der Ansprüche 2 bis 9, **dadurch gekennzeichnet, dass** die interferierende Probe eine Serumprobe und/oder ZSF ist.

11. Set zur Durchführung eines Verfahrens nach einem der Ansprüche 1 bis 10, welches eine biologische Probe, die eine Kynurenin-Aminotransferase (KAT), vorzugsweise in Verbindung mit einer Gewebeprobe oder einem Homogenat, insbesondere einem Leber-Homogenat oder synthetisierter Leber, zweckmäßige Puffer und Kynurenin, vorzugsweise L-Kynurenin aufweist, und gegebenenfalls auch Pyridoxal-5'-phosphat umfasst.

12. Set nach Anspruch 11, **dadurch gekennzeichnet, dass** die Kynurenin-Aminotransferase (KAT) KAT I, KAT II oder KAT III, ist.

13. Set nach Anspruch 11 oder 12, welches weiters eine Oxo-Säure, vorzugsweise ausgewählt aus Pyruvat, 3-Hydroxypyruvat, 2-Oxoglutarat, 2-Oxoisovalerat, 2-Oxoadipat, Phenylpyruvat, 2-Oxobutyrat, Glyoxalat, Oxaloacetat, 2-Oxo-gamma-methiolbutyrat, 2-Oxo-n-valerat, 2-Oxo-n-caproat und 2-Oxoisocaproat, umfasst.

14. Set nach einem der Ansprüche 11 bis 13, welches weiters ein Protein-denaturierendes Mittel, vorzugsweise in einem Mikrozentrifugenröhrchen, umfasst.

15. Set nach einem der Ansprüche 11 bis 14, welches weiters Kynurensäure-, Anthranilsäure- und/oder 3-Hydroxykynurenin-Standards umfasst.

16. Verwendung eines Sets nach einem der Ansprüche 11 bis 15 für ein Verfahren nach einem der Ansprüche 1 bis 10.

## Claims

1. A method for measuring the activity of a kynurenine aminotransferase (KAT), comprising the step of measuring the activity in the presence of an interfering sample selected from a serum sample, and detecting the conversion of kynurenine and/or kynurenic acid and/or anthranilic acid and/or 3-hydroxykynurenine or pyroxidal-5'-phosphate.

2. A method for measuring the activity of a kynurenine aminotransferase (KAT), comprising the step of measuring the activity in the presence of an interfering sample preferably selected from a CSF (cerebrospinal fluid) or serum sample, and detecting the conversion of kynurenine and/or kynurenic acid and/or anthranilic acid and/or 3-hydroxykynurenine or pyroxidal-5'-phosphate, **characterized in that** the activity is determined for two different quantities of the interfering sample and a ratio of these activities is determined.

3. The method as claimed in claim 1 or claim 2, **characterized in that** the kynurenine aminotransferase (KAT) is KAT I, KAT II or KAT III.

4. The method as claimed in claim 1, 2 or 3, **characterized in that** the KAT and/or KAT activity originates from a tissue sample, in particular a tissue homogenate, preferably a liver tissue sample, more preferably an isolated or synthesized liver tissue sample.

5. The method as claimed in claim 4, **characterized in that** the tissue sample originates from a mammal, preferably a rodent or a human being.

6. The method as claimed in one of claims 1 to 5, **characterized in that** the interfering sample originates from a mammal, preferably a human being.

7. The method as claimed in one of claims 1 to 6, comprising the step of comparing the activity with the activity of the KAT, preferably from a tissue sample as defined in claim 4 or claim 5, in the absence of the interfering sample or by using a different quantity of the interfering sample or the enzyme.

8. A method for diagnosing a pathology associated with kynurenine or kynurenic acid metabolism, preferably a neuroimmunological, neuroinflammatory or neurodegenerative pathology, schizophrenia, depression or multiple sclerosis, using the (in-vitro) method as claimed in claims 1 to 7, wherein the pathology is indicated by a reduction in activity of less than 80%, preferably less than 60%, particularly preferably less than 50%, more preferably less than 40%, particularly preferably less than 30%, most preferably less than 20%, compared with the activity without the interfering component (control).

9. The method as claimed in one of claims 1 to 8 for measuring the activity of a KAT, **characterized in that** the conversion of kynurenine and/or kynurenic acid is detected.

10. The method as claimed in one of claims 2 to 9, **characterized in that** the interfering sample is a serum sample and/or CSF.

11. A kit for carrying out a method as claimed in one of claims 1 to 10, comprising a biological sample which includes a kynurenine aminotransferase (KAT), preferably in combination with a tissue sample or a homogenate, in particular a liver homogenate or synthesized liver, appropriate buffers and kynurenine, preferably L-kynurenine, and optionally also comprising pyridoxal-5'-phosphate.

12. The kit as claimed in claim 11, **characterized in that** the kynurenine aminotransferase (KAT) is KAT I, KAT II or KAT III.

13. The kit as claimed in claim 11 or 12, further comprising an oxoacid, preferably selected from pyruvate, 3-hydroxypyruvate, 2-oxoglutarate, 2-oxoisovalerate, 2-oxoadipate, phenylpyruvate, 2-oxobutyrate, glyoxalate, oxaloacetate, 2-oxo-gamma-methiolbutyrate, 2-oxo-n-valerate, 2-oxo-n-caproate, and 2-oxoisocaproate.

14. The kit as claimed in one of claims 11 to 13, further comprising a protein-denaturing agent, preferably in a microcentrifuge tube.

15. The kit as claimed in one of claims 11 to 14, further comprising kynurenic acid, anthranilic acid and/or 3-hydroxykynurenine standards.

16. Use of a kit as claimed in any one of claims 11 to 15, in a method as claimed in one of claims 1 to 10.

## Revendications

1. Procédé de mesure de l'activité d'une kynurénine aminotransférase (KAT) comprenant l'étape de mesure de l'activité en présence d'un échantillon interférant, choisi parmi un échantillon de sérum, et la détection de la transformation de la kynurénine et/ou de l'acide kynurénique et/ou de l'acide anthranilique et/ou de la 3-hydroxykynurénine ou du pyridoxal-5'-phosphate.

2. Procédé de mesure de l'activité d'une kynurénine aminotransférase (KAT) comprenant l'étape de mesure de l'activité en présence d'un échantillon interférant, choisi de préférence parmi un échantillon de liquide céphalorachidien (LCR) ou de sérum, et la détection de la transformation de la kynurénine et/ou de l'acide kynurénique et/ou de l'acide anthranilique et/ou de la 3-hydroxykynurénine ou du pyridoxal-5'-phosphate, **caractérisé en ce que** l'activité est déterminée pour deux quantités différentes de l'échantillon interférant, et qu'un rapport de ces activités est déterminé.

3. Procédé selon la revendication 1 ou 2, **caractérisé en ce que** la kynurénine-aminotransférase (KAT) est la KAT I, la KAT II ou la KAT III.

4. Procédé selon la revendication 1, 2 ou 3, **caractérisé en ce que** l'activité provient de la KAT et/ou de la KAT d'un échantillon de tissu, en particulier un homogénat tissulaire, de préférence un échantillon de tissu hépatique, de manière davantage préférée un échantillon de tissu hépatique isolé ou synthétisé.

5. Procédé selon la revendication 4, **caractérisé en ce que** l'échantillon de tissu provient d'un mammifère, de préférence d'un rongeur ou d'un Homme.

6. Procédé selon l'une quelconque des revendications 1 à 5, **caractérisé en ce que** l'échantillon interférant provient d'un mammifère, de préférence d'un Homme.

7. Procédé selon l'une quelconque des revendications 1 à 6, comprenant l'étape de comparaison de l'activité à l'activité de la KAT, de préférence d'un échantillon de tissu comme décrit dans la revendication 4 ou 5, en l'absence de l'échantillon interférant ou par l'utilisation d'une autre quantité de l'échantillon interférant ou de l'enzyme.

8. Procédé de diagnostic d'une pathologie qui est en relation avec le métabolisme de la kynurénine ou de l'acide kynurénique, de préférence d'une pathologie neuro-immunologique, neuro-inflammatoire ou neuro-dégénérative, d'une schizophrénie, d'une dépression ou d'une sclérose en plaques, moyennant l'utilisation du procédé (*in vitro*) selon les revendications 1 à 7, une réduction de l'activité de moins de 80 %, de préférence de moins de 60 %, de manière particulièrement préférée de moins de 50 %, de manière davantage préférée de moins de 40 %, de manière particulièrement préférée de moins de 30 %, de manière préférée entre toutes de moins de 20 % par rapport à l'activité sans le composant interférant (témoin) indiquant la pathologie.

9. Procédé selon l'une quelconque des revendications 1 à 8 pour la mesure de l'activité d'une KAT, **caractérisé en ce que** la transformation de la kynurénine et/ou de l'acide kynurénique est détectée.

10. Procédé selon l'une quelconque des revendications 2 à 9, **caractérisé en ce que** l'échantillon interférant est un échantillon de sérum ou de LCR.

11. Kit de réalisation d'un procédé selon l'une quelconque des revendications 1 à 10, lequel comporte un échantillon biologique qui contient une kynurénine aminotransférase (KAT), de préférence en liaison avec un échantillon de tissu ou un homogénat, en particulier un homogénat de foie ou de foie synthétisé, des tampons appropriés et de la kynurénine, de préférence de la L-kynurénine, et comprend le cas échéant aussi du pyridoxal-5'-phosphate.

12. Kit selon la revendication 11, **caractérisé en ce que** la kynurénine aminotransférase (KAT) est de la KAT I, de la KAT II ou de la KAT III.

13. Kit selon la revendication 11 ou 12, qui comporte en outre un oxoacide, choisi de préférence parmi un pyruvate, un 3-hydroxypyruvate, un 2-oxoglutarate, un 2-oxo-isovalérate, un 2-oxo-adipate, un phénylpyruvate, un 2-oxobutyrate, un glycoxalate, un oxaloacétate, un 2-oxo-gamma-méthiolbutyrate, un 2-oxo-n-valérate, un 2-oxo-n-caproate et un 2-oxo-isocaproate.

14. Kit selon l'une quelconque des revendications 11 à 13, qui comprend en outre un agent dénaturant de protéine, de préférence dans un microtube pour centrifugeuse.

15. Kit selon l'une quelconque des revendications 11 à 14, qui comprend en outre des solutions étalons d'acide kynurénique, d'acide anthranilique et/ou de 3-hydroxykynurénine.

16. Utilisation d'un kit selon l'une quelconque des revendications 11 à 15 pour un procédé selon l'une quelconque des revendications 1 à 10.
